(19) Europäisches Patentamt
European Patent Office
Office européen des brevets

(11) EP 1 110 948 A2

(12) EUROPEAN PATENT APPLICATION

(43) Date of publication:
**27.06.2001 Bulletin 2001/26**

(51) Int Cl.[7]: **C07C 279/12**, C08K 5/31

(21) Application number: **00403551.5**

(22) Date of filing: **15.12.2000**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **20.12.1999 KR 9959391**

(71) Applicant: **SK Corporation**
**Seoul 110-110 (KR)**

(72) Inventors:
- **Song, Won-Seong**
  **Yusung-ku, Taejon 305-390 (KR)**
- **Lim, Kwang-Min**
  **Yusung-ku, Taejon 305-390 (KR)**
- **Park, Sang-Joon**
  **Yusung-ku, Taejon 305-390 (KR)**
- **Han, Jong-Sok**
  **Yusung-ku, Taejon 305-390 (KR)**

(74) Representative: **Boulinguiez, Didier**
**Cabinet Plasseraud**
**84, rue d'Amsterdam**
**75440 Paris Cedex 09 (FR)**

# (54) Polyhexamethyleneguanidine phosphate powder, method of making the same and antimicrobial resin containing the same

(57) Disclosed is a polyhexamethyleneguanidine phosphate compound, with a molecular weight of 1,000 or greater, represented by the following chemical formula I, which can provide antimicrobial activity to various resins. The antimicrobial agent exhibits superior antimicrobial activity in a variety of resins without deleteriously affecting physical properties of resins and persists in antimicrobial activity for a long period of time in addition to being safe to the body. Its preparation is also disclosed, which is achieved by spray-drying a liquid phase of the polyhexamethyleneguanidine phosphate compound preheated to 70 to 90 °C in a spray dryer whose chamber is maintained at 90 to 110 °C with a rotating plate with small through-holes being operated at a speed of 18,000 to 25,000 rpm. The invention also relates to antimicrobial resins comprising the antimicrobial agent.

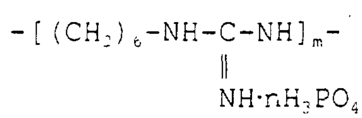

(I)

wherein m is an integer of 4 to 7 and n is an integer of 1 to 14.

EP 1 110 948 A2

**Description**

BACKGROUND OF THE INVENTION

1. Field of the invention

The present invention relates to a polyhexamethyleneguanidine phosphate powder. More particularly, the present invention relates to a polyhexamethyleneguanidine phosphate powder which can be applied for resin processing, thereby allowing the resin products to have antimicrobial activity. Also, the present invention is concerned with a method for preparing a polyhexamethyleneguanidine phosphate powder and with antimicrobial resins containing the same.

2. Description of the Prior Art

The great advances which have been achieved in plastic technology enables plastics to be applied for a variety of fields. Recently increased interest in health and hygiene has been infallibly reflected into the plastic industry, developing various products with antimicrobial functions. The antimicrobial activity of plastic products are usually achieved by the treatment with biocides. To this end, active research has been directed to the development of antimicrobial agents which are easy to process along with resins as well as perform desirable antimicrobial functions.

Conventional antimicrobial agents addible to resins are largely divided into two groups: organic and inorganic antimicrobial agents. In most cases, inorganic antimicrobial agents, which are usually based on metals such as silver or copper, are employed by virtue of their durability to high temperature conditions of resin processes. Inorganic antimicrobial agents, however, are not so widely used owing to the disadvantages as follows. For example, a large quantity of inorganic antimicrobial agents are required because of their weaker antimicrobial activity than that of organic agents. Also, they show relatively poor activity against fungi. In addition to being relatively expensive, they demand difficult conditions for their being dispersed. Further, the metal ions of inorganic antimicrobial agents cause discoloration during or after the processing.

In contrast, organic antimicrobial agents are relatively inexpensive and show good activity against microbes at small amounts. However, organic antimicrobial agents have serious disadvantages. They are decomposed at the processing temperatures of resins and lose their antimicrobial activity. Furthermore, they cause discoloration after processing and their activity does not persist for a long period of time because they easily elute into the surface of products. Accordingly, these disadvantages limit organic antimicrobial agents to a very narrow spectrum of uses.

With so good processability, benzimidazole-based antimicrobial agents can be used in plastic processing, but have relatively poor activity against bacteria and may discolor the resins after processing. Superior as it is in antimicrobial activity and processability, 10, 10-oxybisphenoxyarsine is inclined to decrease in its consumed amount because it is liable to decompose to UV light and its arsenic component may cause a problem of safety.

SUMMARY OF THE INVENTION

Leading to the present invention, the intensive and thorough research on antimicrobial agents useful for plastics, repeated by the present inventors aiming to surmount the problems encountered in prior arts, resulted in the finding that polyhexamethyleneguanidine phosphate (hereinafter referred to as "PHMG") powder with a molecular weight of 1,000 or greater is not decomposed in processing conditions of various resins, shows high dispersibility, causes no discoloration, and rarely elutes into the surface of the resin products, thereby maintaining antimicrobial persistency for a long period of time.

Therefore, it is an object of the present invention to provide a PHMG powder which is useful as an antimicrobial agent in a variety of synthetic resins as well as superior in antimicrobial activity and persistency.

It is another object of the present invention to provide a method for preparing such a PHMG powder.

It is a further object of the present invention to provide an antimicrobial resin containing the PHMG as an antimicrobialally active ingredient.

In accordance with an embodiment of the present invention, there is provided a method for preparing a PHMG powder, comprising the steps of pre-heating at 70 to 90 °C a liquid phase of PHMG compound with a molecular weight of 1,000 or greater, represented by the following chemical formula I:

$$-[(CH_2)_6-NH-\underset{\underset{NH\cdot nH_3PO_4}{\|}}{C}-NH]_m- \qquad (I)$$

wherein m is an integer of 4 to 7 and n is an integer of 1 to 14, and spray-drying the liquid phase in a spray dryer whose chamber is maintained at a temperature of 90 to 110 °C with a rotating plate with small through-holes being operated at a speed of 18,000 to 25,000 rpm.

In accordance with another embodiment of the present invention, there is provided such a PHMG powder.

In accordance with a further embodiment of the present invention, there is provided an antimicrobial resin, comprising the PHMG powder at an amount of 0.07 to 1.2 weight % as an antimicrobially active ingredient and a resinous component at the remaining amount to 100 weight % as a base material.

## DETAILED DESCRIPTION OF THE INVENTION

The present invention pertains to PHMG with a weight average molecular weight of 1,000 or greater, represented by the following chemical formula I:

$$-[(CH_2)_6-NH-\underset{\underset{NH\cdot nH_3PO_4}{\|}}{C}-NH]_m- \qquad (I)$$

wherein m is an integer of 4 to 7 and n is an integer of 1 to 14. In contrast to other antimicrobial agents, PHMG has such a structure that it cannot slip out of the structures of plastics when they are cured after the PHMG is compounded with the plastics. In addition, its surprising stability to heat, light, and pH conditions makes the polymeric compound undergo less influences physically and chemically upon processing, persisting in antimicrobial activity for a long period of time.

Since its liquid phase cannot be applied to plastic processing, the PHMC of the present invention must be converted from the liquid phase of its produced state to a powdered phase through a spray drying process. In detail, a liquid phase of PHMG is preheated to 70 to 90 °C in a separate chamber. When the temperature is below 70 °C, it takes a long period of time when hot air drying in a subsequent step. On the other hand, when it exceeds 90 °C, moisture is evaporated to cause pre-precipitation. Next, the preheated liquid phase is fed to a spray dryer, after which the liquid phase is allowed to pass through the spray dryer while a rotating plate with small through-holes is operated at a speed of 18,000 to 25,000 rpm. When the high-speed rotating plate. of the spray dryer is rotated at a speed less than 18,000 rpm, the resulting particles become large. On the other hand, a rotating speed greater than 25,000 rpm deteriorates the powering efficiency. Then, hot air drying evaporates moisture from the PHMG. In this regard, the dryer chamber is maintained at 90 to 110 °C. When the dryer chamber is maintained at a temperature less than 90 °C, a long period of time is required to give the powder. On the other hand, a temperature higher than 110 °C causes the evaporation to be conducted at a faster rate than that of the powdering.

The PHMG powder thus obtained can be used as an antimicrobial agent for resin processing. Its excellent processability enables PHMG powder to be applied to a variety of resins, including polypropylene (hereinafter referred to as "PP"), polyethylene (hereinafter referred to as "PE"), polystyrene (hereinafter referred to as "PS"), polyvinyl chloride (hereinafter referred to as "PVC"), acrylonitrilebutadiene styrene (hereinafter referred to as "ABS"), and polyester, to produce antimicrobial plastic products. For use, the PHMG powder can be formulated, together with a pellet of a resinous component, for example, PP, PE, PS, PVC, ABS, and polyester, at an amount of 3 to 20 wt% in a master batch (hereinafter referred to as "M/B") and more preferably at an amount of 10 to 15 wt%. When a M/B with a content of 10 wt% of the antimicrobial agent is added at an amount of 3 wt% to a resinous component, the final plastic products processed are to contain the antimicrobial agent at an amount of 0.3 wt%. As for PP or PE products, their content of the antimicrobial agent preferably ranges from 0.07 to 1.2 wt% and more preferably from 0.2 to 1.0 wt%. For example, if the final content of the antimicrobial agent is below 0.07 wt%, the product is poor in antimicrobial activity. On the other hand, a final content greater than 1.2 wt% of the antimicrobial agent deteriorates the physical properties of the plastic products.

For the processing of PS, PVC and polyesters, the PHMG powder may be added at an amount of 0.08 to 1.0 wt% and preferably at an amount of 0.2 to 0.8 wt%. For example, a final plastic product with an antimicrobial content less than

0.08 wt% shows poor antimicrobial activity. On the other hand, when the content of the antimicrobial agent exceeds 1.0 wt%, there may occur an influence on the physical properties of the final products.
For ABS, the PHMG powder may be used at an amount of 0.08 to 0.8 wt% and preferably at an amount of 0.2 to 0.7 wt%. For example, the final ABS products show poor antimicrobial activity at a content of less than 0.08 wt% of the antimicrobial agent and may be deteriorated in physical property at a content of greater than 0.8 wt% of the antimicrobial agent.

A better understanding of the present invention may be obtained in light of the following examples which are set forth to illustrate, but are not to be construed to limit the present invention.

EXAMPLE 1

Preparation of PHMG Powder

A PHMG powder was obtained from a liquid phase under the condition of Table 1, below, by means of a spray drying process

TABLE 1

| Conditions for Spray Drying | |
|---|---|
| Items | Conditions |
| Preheating Temp. | 70 to 80°C |
| Rotating Plate Speed | 22,000 rpm |
| Drying Chamber Temp. | 95 to 100°C |
| Production Rate | 40 kg/hr |

A liquid PHMG preheated as indicated in Table 1 was fed to an upper portion of a spray drying chamber, so as to finally afford a yellowish white PHMG powder which was very fine and did not conglomerate and scorch with a moisture content of 2 % or less.

EXAMPLE 2

Preparation of Antimicrobial PP M/B

PP pellets mixed with 10 wt% of the PHMG powder prepared in Example 1 were fed into an extruder and processed under the condition indicated in Table 2, below. Preparation of an antimicrobial PP M/B was normally achieved with occurrence of no problems attributed to the feeding of the antimicrobial agent.

TABLE 2

| Processing Condition of Antimicrobial PP M/B | |
|---|---|
| Items | Conditions |
| Equip. Model | Twin Screw Extruder L/D:36 |
| Screw Temp. °C | 160/170/180/180/190/190/190/195/195/195/195 (°C) |
| Screw Speed | 220 rpm |
| Feeding Rate | 18.5 kg/hr |
| Resin Temp. | 220°C |

EXAMPLE 3

Preparation of Antimicrobial Fiber by Spinning

A fiber product was prepared by spinning the antimicrobial PP M/B obtained in Example 2, under the condition shown in Table 3, below. Along with PP pellets, the antimicrobial PP M/B was fed at an amount of 3 wt%, 5 wt% and 10 wt% based on the total weight fed. During the production of fibers under the above condition, there occurred no problems of cutting yarns or clogging spinnerets with residues. Merely when the final concentration of the antimicrobial agent was increased over 1.0 wt% (that is, the M/B was fed more than an amount of 10 wt%), the fiber products were

occasionally found to show weak strength. At a less concentration, no problems occurred for the processability of fibers.

TABLE 3

| Processing Conditions for Spinning of Antimicrobial PP Fiber | |
|---|---|
| Items | Conditions |
| Screw Temp. | 180/220/220/235/245/245 (°C) |
| Screw Speed | 77rpm |
| Roller 1/Roller 2 Temp | 50/80 (°C) |
| Roller 1/Roller 2/Winder | 200/600/580(meter/min) |

EXAMPLE 4

Preparation of Antimicrobial PE M/B

PE pellets mixed with 10 wt% of the PHMG powder prepared in Example 1 were fed into an extruder and processed under the condition indicated in Table 4, below. Preparation of an antimicrobial PE M/B was normally achieved with occurrence of no problems attributed to the feeding of the antimicrobial agent.

TABLE 4

| Processing Condition for Antimicrobial PE M/B | |
|---|---|
| Items | Conditions |
| Screw Temp. | 170/170/165/175/175/175/180/180/180/185/190 (°C) |
| Screw (°C) | 207 rpm |
| Feed Rate | 30.0 kg/hr |
| Resin Temp. | 212 °C |

EXAMPLE 5

Evaluation for Antimicrobial Activity in PP Fiber

An evaluation for antimicrobial activity was made of the PP fiber produced in Example 3. The test of antimicrobial activity was conducted according to the shake flask method of Association of Antibacterial Treatments for Textiles.

0.75 g of each of samples taken from the fibers with various contents of the antimicrobial agent was put in a flask containing 70 ml of sterile phosphate buffered saline solution, which was then inoculated with 5 ml of pre-cultured *Staphylococcus aureus* at a density of $10^{4\text{-}5}$ cfu/ml. After being cultured for 1 hour with shaking at 320 rpm, the bacterial solutions were spread over media in a serial dilution method and cultured for 18 hours at 37 °C, followed by counting the colonies grown. By comparing the numbers of the bacterial colonies formed on the media upon the treatment with and without the bacterial agent, bacteria reduction percentages were calculated according to the following equation.

$$\%\ Bacteria\ Reduction = \frac{(A-B)}{A} \times 100$$

wherein A represents the number of the bacterial colonies grown on the media containing the fiber treated without antimicrobial agents and B represents the number of the bacterial colonies grown on the media containing the antimicrobially treated fiber. The results are given in Table 5, below.

TABLE 5

| Antimicrobial Activity in PP Fibers | | | |
|---|---|---|---|
| | Fiber Samples | | |
| | Non-treated | 0.3% PHMG treated | 0.5% PHMG treated |
| Cell No. (cfu/ml) | $5.0 \times 10^5$ | 5 | 5 |

TABLE 5 (continued)

| Antimicrobial Activity in PP Fibers | | | |
|---|---|---|---|
| | Fiber Samples | | |
| | Non-treated | 0.3% PHMG treated | 0.5% PHMG treated |
| Reduction Rate (%) | - | >99.9 | >99.9 |

As apparent in Table 5, the antimicrobial resins of the present invention are superior in antimicrobial activity, showing a bacteria reduction percentage of as large as 99.9 % or greater. These results demonstrate that PHMG can be processed in its integrity without losing its antimicrobial activity even under the processing condition of as high as 245 °C when it is formulated into the M/B or spun in fibers.

EXAMPLE 6

Test of Antimicrobial PP Fiber for Durability to Water (Washability)

Generally, as explained previously, organic antimicrobial agents are difficult to process into resins in addition to being poor in the persistency of antimicrobial activity because of their easy elution into surfaces although they are processed into resins. Where they are brought into contact with water, more serious elution occurs.

A PHMG-containing antimicrobial PP fiber obtained in Example 3 was tested for water resistance in a washing method.

In this regard, the antimicrobial PP fiber with a content of 0.5 wt% of the PHMG powder was used as a test sample while a PP film containing 0.5 wt% of 2-n-octyl-4-isothiazoline-3-one, which was a conventional antimicrobial agent applicable to resin processing, served as a control. Washing was conducted at about 43 °C in accordance with L 0217-103 of Japanese Industrial Standard (JIS) (this washing test is conducted under a far severer condition than an ordinary environment in which it comes into contact with water). After completion of the washing, the samples were tested for antimicrobial activity against *Staphylococcus aureus.* Given in Table 6, below, are the antimicrobial activity results according to the rounds of the washing.

TABLE 6

| Antimicrobial Activity Change According to Rounds of Washing | | |
|---|---|---|
| Rounds of Washing | Bacteria Reduction Rate (%) of PP Fibers | |
| | PHMG PHMG | OIT OIT* |
| 0 | >99.9 | >99.9 |
| 10 | 99.7 | 76.5 |
| 20 | 83.7 | 55.4 |
| 30 | 79.2 | <30.0 |
| 40 | 78.7 | <30.0 |

note:* 2-n-octyl-4-isothiazoline-3-one

After 40 rounds of the washing, as recognized from the data of Table 6, the plastic product treated with PHMG retained antimicrobial activity at a level of about 80 % of the original one, demonstrating that PHMG rarely eluted into the surface of the product, but was of potent water resistancy, that is, high antimicrobial persistency. On the contrary, the conventional antimicrobial agent was found to considerably slip out of the fiber as the antimicrobial activity of the control PP fiber significantly decreased with an increase of the rounds of washing.

EXAMPLE 7

Evaluation for Antimicrobial Activity in PE Product

An evaluation for antimicrobial activity was made of a thin PE film (used as a package material for hygienic products) with a content of 0.5 wt% of the PHMG powder. The test of antimicrobial activity was conducted according to the shake flask method of SEK.

0.75 g of each of the film samples was put in a flask containing 70 ml of sterile phosphate buffered saline

solution, which was then inoculated with 5 ml of pre-cultured *Staphylococcus aureus* at a density of $10^{4-5}$ cfu/ml. After being cultured for 1 hour with shaking at 320 rpm, the bacterial solutions were spread over media in a serial dilution method and cultured for 18 hours at 37 °C, followed by counting the colonies grown. By comparing the numbers of the bacterial colonies formed on media upon treatment with and without the antimicrobial agent, bacteria reduction percentages were calculated. The results are given in Table 7, below.

TABLE 7

| Antimicrobial Activity in PE Films | | |
|---|---|---|
| | PE Films | |
| | Non-treated | Treated with PHMG |
| Cell No. (cfu/ml)<br>Reduction Rate (%) | $4.4 \times 10^4$<br>- | 2<br>>99.9 |

As apparent in Table 7, the antimicrobial resins of the present invention are superior in antimicrobial activity, showing a bacteria reduction percentage of as large as 99.9 % or greater. These results demonstrate that PHMG can be processed in its integrity without losing its antimicrobial activity under the processing condition of a film.

EXAMPLE 8

Antimicrobial Effect in PS Product

Plastic cups with final contents of 0.3 and 0.5 wt% of the PHMG powder were made from PS. These PS cup samples were tested for antimicrobial activity in accordance with the shake flask method of SEK.

0.75 g of each of the samples taken from the PS cups with contents of the antimicrobial agent was put in a flask containing 70 ml of sterile phosphate buffered saline solution, which was then inoculated with 5 ml of pre-cultured *Staphylococcus aureus* at a density of $10^{4-5}$ cfu/ml. After being cultured for 1 hour with shaking at 320 rpm, the bacterial solutions were spread over media in a serial dilution method and cultured for 18 hours at 37 °C, followed by counting the colonies grown. By comparing the numbers of the bacterial colonies formed on the media upon the treatment with and without the antimicrobial agent, bacteria reduction percentages were calculated. The results are given in Table 8, below.

Table 8

| Antimicrobial Activity in PS Products | | | |
|---|---|---|---|
| | PS Samples | | |
| | Non-treated | 0.3% PHMG Treated | 0.5% PHMG Treated |
| (cfu/ml)<br>(%) | $4.3 \times 10^4$<br>- | 0<br>>99.9 | 0<br>>99.9 |

As apparent in Table 8, a content of 0.3 wt% or greater of the antimicrobial agent guarantees PS products to have superior antimicrobial activity, resulting in a bacteria reduction percentage of as large as 99.9 % or greater. These results demonstrate that PHMG can be processed in its integrity without losing its antimicrobial activity under the processing condition of PS.

EXAMPLE 9

Antimicrobial Effect in PVC Product

With contents of 0.3 and 0.4 wt% of the PHMG powder, base layers of tile carpets were made from PVC. These PVC samples were tested for antibacterial activity and antifungal activity.

A film contact method was used for the antibacterial test of the PVC samples. 100 µl of a pre-cultured *Staphylococcus aureus* solution was put on each of the PVC samples which were then covered with parafilm, followed by culturing the bacteria for 18 hours at 37 °C. After removing the parafilm, the bacteria were recovered in phosphate buffered saline solutions and spread over media by a serial dilution method. They were cultured for 24 hours at 37 °C, after which the appearing colonies were counted. By comparing the numbers of the bacterial colonies formed on the

media upon the treatment with and without the antimicrobial agent, bacteria reduction percentages were calculated. The results are given in Table 9, below.

TABLE 9

| Antimicrobial Activity in PVC Products | | | |
|---|---|---|---|
| | PVC Samples | | |
| | Non-created | 0.3% PHMG Treated | 0.4% PHMG Treated |
| Cell No. (cfu/ml)<br>Reduction Rate(%) | $2.4 \times 10^6$<br>- | 0<br>>99.9 | 0<br>>99.9 |

As apparent in Table 9, a content of 0.3 wt% or greater of the PHMG allows the PVC product to have superior antibacterial activity, from which the PHMG was also found to be effective for PVC processing.

An examination was made of antifungal activity. In this regard, a PVC sample with a diameter of about 2 cm was put on the center of the bottom of a sterile petri dish into which liquified potato-dextrose agar containing a mixture of two fungal spores *(Aspergillus niger* and *Penicillium funiculosum)* was then poured. After the agar was cooled at room temperature, the fungi were allowed to grow for seven days while an observation was made as to the occurrence and size of a growth inhibition halo zone. The results are given in Table 10, below.

TABLE 10

| Antifungal Activity in PVC Products | | |
|---|---|---|
| Strains | PVC Samples | |
| | 0.3% PHMG Treated | 0.4% PHMG Treated |
| *A.* niger<br>*P. funiculosm* | 2.5<br>1.5 | 3.3<br>3.3 |
| * mm<br>* Numbers=(Diameter of halo zone-Diameter of Sample)/2 | | |

As shown in Table 10, a growth inhibition halo was formed around each of the test samples, demonstrating that the PHMG is superior in antifungal activity. Therefore, the antimicrobial PHMG can be applied for PVC products which are exposed to the contamination with fungi as well as bacteria.

EXAMPLE 10

Antimicrobial Effect in ABS Product

There were made ABS products which comprised 0.2, 0.4 and 0.6 wt% of the PHMG powder. These ABS samples were tested for antimicrobial activity in accordance with a film contact method. 100 µl of a pre-cultured *Staphylococcus aureus* solution was put on each of the ABS samples which were then covered with parafilm, followed by culturing the bacteria for 18 hours at 37 °C. After removing the parafilm, the bacteria were recovered in phosphate buffered saline solutions and spread over media by a serial dilution method. They were cultured for 24 hours at 37 °C, after which the appearing colonies were counted. By comparing the numbers of the bacterial colonies formed on the media upon the treatment with and without the antimicrobial agent, bacteria reduction percentages were calculated. The results are given in Table 11, below.

TABLE 11

| Antimicrobial Activity in ABS Products | | | | |
|---|---|---|---|---|
| | Non- Treated | Antimicrobial Agent Treated | | |
| | | 0.2% | 0.4% | 0.6% |
| Reduction Rate(%) | - | 88.5 | 91.1 | 95.5 |

As apparent in Table 11, a content of 0.4 wt% or greater of the PHMG allows ABS products to have superior antimicrobial activity without deleteriously affecting the impact strength of ABS, from which the PHMG was also found

to be effective for ABS processing, which is usually conducted at 220 °C.

EXAMPLE 11

Test of Antimicrobial PE and ABS Products for Durability to Water (Washability)

In order to examine whether the washability of the PHMG is obtained in other resins as described in Example 6, PE and ABS products containing the antimicrobial compound of the present invention **were** tested as follows.
In this regard, an antimicrobial PE film containing 0.5 wt% of PHMG was used along with a control of an PE film containing 2-n-octyl-4-isothiazoline-3-one. As for ABS, the PHMG powder was used at an amount of 0.6 wt% while an inorganic antimicrobial, such as Novaron, produced by Toagosei Co. Ltd., Japan, was used at an amount of 1.0 wt% as a control. For testing, the PE film samples were sliced and the ABS samples were chopped.
Washing was conducted in accordance with L 0217-103 of Japanese Industrial Standard (JIS) as mentioned in Example 6. After completion of the washing, the samples were tested for antimicrobial activity against *Staphylococcus aureus.* Given in Tables 12 and 13, below, are the antimicrobial activity results according to the rounds of the washing.

TABLE 12

| Antimicrobial Activity Change in PE Product According to Rounds of Washing | | |
|---|---|---|
| Rounds of Washing | Bacterial Reduction Rate (%) | |
| | Treated with PHMG | Treated with OIT* |
| 0 | >99.9 | >99.9 |
| 10 | 99.0 | 74.8 |
| 20 | 87.1 | 63.1 |
| 30 | 75.6 | <30.0 |
| 40 | 74.9 | <30.0 |

note:* 2-n-octyl-4-isothiazoline-3-one

After 40 rounds of the washing, as recognized from the data of Table 12, the PE product treated with PHMG retained antimicrobial activity at a level of about 75 % of the original one, which demonstrated that it was difficult for PHMG to elute into the surface of the product. On the contrary, the conventional antimicrobial agent was found to considerably slip out of the fiber as the antimicrobial activity of the control PP fiber significantly decreased with an increase of the rounds of washing.

TABLE 13

| Antimicrobial Activity Change in ABS Product According to Rounds of Washing | | |
|---|---|---|
| Rounds of Washing | Bacterial Reduction Rate (%) | |
| | Treated with PHMG | Treated with Novaron* |
| 0 | 96.4 | 91.1 |
| 10 | 93.6 | 87.4 |
| 20 | 88.8 | 84.5 |
| 30 | 80.5 | 81.9 |
| 40 | 77.9 | 78.8 |

note:* 2-n-octyl-4-isothiazoline-3-one

For comparison of the antimicrobial activity in ABS, the inorganic antimicrobial agent was employed because none of other organic antimicrobial agents were found to be applied to ABS. As recognized in Table 13, the obtained water resistancy of the PHMG in ABS was similar to that in PP or PE. Showing a similar antimicrobial behavior to that of PHMG, the inorganic antimicrobial agent was found to be good in washability. In result, PHMG is not willing to elute into the surface of ABS products, exhibiting an antimicrobial effect similar to that of the inorganic agent.

EXAMPLE 12

Antimicrobial Effect in Polyester Product

Polyester decoration products (various shapes, but not in fiber form) with a content of 0.5 wt% of the PHMG powder were tested for antimicrobial activity according to the shake flask method of SEK.

0.75 g of each of the product samples was put in a flask containing 70 ml of sterile phosphate buffered saline solution, which was then inoculated with 5 ml of pre-cultured *Staphylococcus aureus* at a density of $10^{4-5}$ cfu/ml. After being cultured for 1 hour with shaking at 320 rpm, the bacterial solutions were spread over media in a serial dilution method and cultured for 18 hours at 37 °C, followed by counting the colonies grown. By comparing the numbers of the bacterial colonies formed on media upon treatment with and without the antimicrobial agent, bacteria reduction percentages were calculated. The results are given in Table 14, below.

TABLE 14

| Antimicrobial Activity in Polyester Products | | | | | | | | |
|---|---|---|---|---|---|---|---|---|
| | Non- treated | Polyester Products Treated with PHMG | | | | | | |
| | | A-1 | A-2 | A-3 | B-1 | B-2 | C-1 | C-2 |
| Reduction Rate(%) | - | >99.9 | >99.9 | 99.7 | >99.9 | >99.9 | >99.9 | >99.9 |

As apparent in Table 14, a content of 0.5 wt% of the antimicrobial agent guarantees polyester products to have superior antimicrobial activity, resulting in a bacteria reduction percentage of as large as 99.7 % or greater. These results demonstrate that PHMG can be processed in its integrity without losing its antimicrobial activity under the processing condition of polyester.

As described hereinbefore, the PHMG of the present invention has various advantages over conventional resin-applicable inorganic antimicrobial agents, which show problems of being expensive and difficult to disperse upon processing, causing discoloration after resin processing, and showing poor antifungal activity, as well as over conventional resin-applicable organic antimicrobial agents, which suffer from the problems of being limited in applicable resin ranges and ready to elute out of products, causing discoloration and decreasing in antimicrobial activity after resin processing. The antimicrobial agent of the present invention exhibits superior antimicrobial activity in a variety of resins without deleteriously affecting physical properties of resins and persists in antimicrobial activity for a long period of time in addition to being safe to the body.

The present invention has been described in an illustrative manner, and it is to be understood that the terminology used is intended to be in the nature of description rather than of limitation. Many modifications and variations of the present invention are possible in light of the above teachings. Therefore, it is to be understood that within the scope of the appended claims, the invention may be practiced otherwise than as specifically described.

## Claims

1. A method for preparing a polyhexamethyleneguanidine phosphate powder, in which a liquid phase of polyhexamethyleneguanidine phosphate compound with a molecular weight of 1,000 or greater, represented by the following chemical formula I:

$$-[(CH_2)_6-NH-\underset{\underset{NH\cdot nH_3PO_4}{\|}}{C}-NH]_m- \qquad (I)$$

wherein m is an integer of 4 to 7 and n is an integer of 1 to 14, is preheated to 70 to 90 °C and spray-dried in a spray dryer whose chamber is maintained at 90 to 110 °C with a rotating plate with small through-holes being operated at a speed of 18,000 to 25,000 rpm.

2. A polyhexamethyleneguanidine phosphate powder, prepared according to the method of claim 1.

3. An antimicrobial resin, comprising the polyhexamethyleneguanidine phosphate powder of claim 2 at an amount of 0.07 to 1.2 weight % as an antimicrobially active ingredient and a resinous component at the remaining amount to 100 weight % as a base material.

4. The antimicrobial resin as set forth in claim 3, wherein said resinous component is selected from the group consisting of polypropylene, polyethylene, polystyrene, polyvinyl chloride, polyester and acrylcnitrilebutadiene styrene.

5. The antimicrobial resin as set forth in claim 4, wherein said polyhexamethyleneguanidine phosphate powder is present at an amount of 0.2 to 1.0 weight % when said resinous component is polypropylene or polyethylene.

6. The antimicrobial resin as set forth in claim 4, wherein said polyhexamethyleneguanidine phosphate powder is present at an amount of 0.08 to 1.0 weight % when said resinous component is polystyrene, polyvinyl chloride or polyester.

7. The antimicrobial resin as set forth in claim 4, wherein said polyhexamethyleneguanidine phosphate powder is present at an amount of 0.08 to 0.8 weight % when said resinous component is acrylonitrilebutadiene styrene.

8. The antimicrobial resin as set forth in claim 3, wherein said polyhexamethyleneguanidine phosphate powder is added to the resinous component as a form of a master batch comprising said polyhexamethyleneguanidine phosphate powder at an amount of 3 to 20 weight% based on total weight of the master batch and a pellet of a resinous component at the remaining amount to 100 weight %.

9. The antimicrobial resin as set forth in claim 8, wherein said resinous component is selected from the group consisting of polypropylene, polyethylene, polystyrene, polyvinyl chloride, polyester and acrylonitrilebutadiene styrene.

10. The antimicrobial resin as set forth in claim 9, wherein said polyhexamethyleneguanidine phosphate powder is present at an amount of 0.2 to 1.0 weight % based on the total weight of the antimicrobial resin when said resinous component is polypropylene or polyethylene.

11. The antimicrobial resin as set forth in claim 9, wherein said polyhexamethyleneguanidine phosphate powder is present at an amount of 0.08 to 1.0 weight % based on the total weight of the antimicrobial resin when said resinous component is polystyrene, polyvinyl chloride or polyester.

12. The antimicrobial resin as set forth in claim 9, wherein said polyhexamethyleneguanidine phosphate powder is present at an amount of 0.08 to 0.8 weight % based on the total weight of the antimicrobial resin when said resinous component is acrylonitrilebutadiene styrene.